# EUROPEAN PATENT SPECIFICATION

(11) **EP 2 011 518 B1**
(45) Date of publication and mention of the grant of the patent: **18.06.2014**
(21) Application number: 07741475.3
(22) Date of filing: 12.04.2007
(51) Int. Cl.: A61K 49/00, C07D 239/46, G01N 33/497

(54) **TEST AGENT FOR DIAGNOSING DYSPEPSIA**
TESTMITTEL ZUR DIAGNOSE VON VERDAUUNGSSTÖRUNGEN
AGENT DE TEST POUR DIAGNOSTIQUER LA DYSPEPSIE

(30) Priority: 13.04.2006 JP 2006111382
(43) Date of publication of application: 07.01.2009
(73) Proprietor: Otsuka Pharmaceutical Co., Ltd., Tokyo 101-8535 (JP)
(72) Inventor: INADA, Makoto, Kawauchi-cho, Tokushima 7710182 (JP); KUNIZAKI, Jun-ichi, Kawauchi-cho, Tokushima 7710182 (JP); IKEI, Nobuhiro, Tokushima-shi, Tokushima 770-0852 (JP); NATSUME, Kuniaki, Tokushima 7710220 (JP)
(74) Representative: HOFFMANN EITLE
(86) International application number: PCT/JP2007/058039
(87) International publication number: WO 2007/119771

(56) References cited:
- WO-A1-97/35622
- WO-A1-02/072153
- WO-A1-2007/013409
- MATTISON LORI K ET AL: "The uracil breath test in the assessment of dihydropyrimidine dehydrogenase activity: pharmacokinetic relationship between expired 13CO2 and plasma [2-13C]dihydrouracil.", CLINICAL CANCER RESEARCH : AN OFFICIAL JOURNAL OF THE AMERICAN ASSOCIATION FOR CANCER RESEARCH 15 JAN 2006 LNKD- PUBMED:16428499, vol. 12, no. 2, 15 January 2006 (2006-01-15), pages 549-555, XP009145025, ISSN: 1078-0432
- BRADEN ET AL: "C-breath tests: Current state of the art and future directions", DIGESTIVE AND LIVER DISEASE, W.B. SAUNDERS, vol. 39, no. 9, 11 August 2007 (2007-08-11), pages 795-805, XP022187791, ISSN: 1590-8658, DOI: DOI:10.1016/J.DLD.2007.06.012
- SUGIYAMA ERIKA ET AL: "Desirable pharmacokinetic properties of (13)C-uracil as a breath test probe of gastric emptying in comparison with (13)C-acetate and (13)C-octanoate in rats.", SCANDINAVIAN JOURNAL OF GASTROENTEROLOGY 2009 LNKD- PUBMED:19585373, vol. 44, no. 9, 2009, pages 1067-1075, XP009145039, ISSN: 1502-7708
- KUBO H ET AL: "Enhancement of oral bioavailability and pharmacological effect of 1-(3,4- dimethoxyphenyl)-2,3-bis(methoxycarbonyl)- 4-hydroxy-6,7,8- trimethoxynaphthalene (TA-7552), a new hypocholesterolemic agent, by micronization in co-ground mixture with D-mannitol", BIOLOGICAL & PHARMACEUTICAL BULLETIN (OF JAPAN), PHARMACEUTICAL SOCIETY OF JAPAN, TOKYO, JP, vol. 19, no. 5, 1 January 1996 (1996-01-01), pages 741-747, XP002493448, ISSN: 0918-6158
- INADA M. ET AL.: 'Relationships among plasma [2-13C]uracil concentrations, breath 13CO2 expiration, and dihydropyrimidine dehydrogenase (DPD) activity in the liver in normal and DPD-deficient dogs' DRUG METAB. DISPOS. vol. 33, no. 3, 2005, pages 381 - 387, XP003018557
- SASAKI Y.: 'Koki Kensa ni Okeru Antei Doitai Riyo' NIPPON ISOTOPE HOSHASEN SOGO KAIGI HOBUNSHU vol. 18TH, 1988, pages 610 - 617, XP003018558
- MARIANI G. ET AL.: 'Radionuclide gastroesophageal motor studies' J. NUCL. MED. vol. 45, no. 6, 2004, pages 1004 - 1028, XP003018559

## Description

### TECHNICAL FIELD

The present invention relates to a composition for use in a method for measuring the gastric-emptying function, which can be used to effectively diagnose dyspepsia, and a method for measuring the gastric-emptying function. Specifically, the present invention relates to a composition that can be used to measure reductions of the gastric-emptying function in a noninvasive manner using expired air, and a method for measuring the gastric-emptying function using the composition. The present invention also relates to a composition for use in a method for diagnosing dyspepsia and a method for diagnosing dyspepsia. Furthermore, the present invention relates to a method for measuring the drug efficiency and treatment effects of a gastrointestinal medicine, in particular, a medicine affecting the alimentary tract motor function on a patient suffering from gastrointestinal disorders caused by dyspepsia and like insufficiencies in the gastric-emptying function.

### BACKGROUND ART

Nowadays, we live in a society with much stress due to rapid and drastic changes in various matters. Accordingly, there has been an increase in patients who complain of gastroenteric pain and discomfort (such as epigastralgia/epigastric discomfort, heavy stomach, heartburn, epigastric fullness, early satiety, nausea, vomiting, etc.). Even if a subject suffers from such symptoms for a long period of time, if no disorders are detected by magendurchleuchtung, endoscopy or abdominal ultrasonography, such symptoms would be diagnosed as unidentified complaints in the abdominal region and would not be subjected to medical treatment, or diagnosed and treated as chronic gastritis.

However, it has recently been considered that such symptoms may be attributable to dysfunctions of the alimentary tract. In the United States, when unidentified complaints last at least four weeks in spite of the absence of structural abnormalities when checked with an endoscope, such conditions are referred to as "symptoms of non-ulcer dyspepsia in the alimentary tract" (Non-ulcer Dyspepsia: NUD, hereunder referred to as "dyspepsia") and distinguished from chronic gastritis (1987, the American Gastroenterological Association).

Dyspepsia is roughly categorized into the following four groups according to subjective symptoms:
(1) a gastroesophageal reflux type (heartburn, acid reflux, reflux feeling, belching), (2) a dysmotility type (early satiety, bloating, anorexia, nausea, vomiting), (3) an ulcer type (night pain, hunger pain, periodic discomfort, abdominal pain), and (4) a non-specific type (symptoms not applicable to (1) to (3) above). These symptoms excluding (1), i.e., the symptoms of (2) to (4), are collectively called Functional Dyspepsia: FD (see, ROME II scale: Non-Patent Document 1). Among these dyspepsias, in particular, the dysmotility type (2) is the most common, and it accounts for 30 to 40% of the total dyspepsias (Non-Patent Document 2).

Treatment of dyspepsia is often conducted as a symptomatic therapy using medicines. Since the gastroesophageal reflux type (1) is significantly related to gastric acid, a medicine that can control the secretion of gastric acid is used. The non-specific type (4) is assumned to be caused by psychological factors, and therefore antianxiety medicines and antidepressants are often used. However, as shown below, medicines for improving the alimentary tract motor function serve a key role in treating these symptoms:
(1) gastroesophageal reflux type: acid antisecretory, antacid, and medicines for improving the alimentary tract motor function;
(2) dysmotility type and (3) ulcer type: medicines for improving alimentary tract motor function; and
(4) non-specific type: medicines for improving the alimentary tract motor function, antianxiety medicines, and antidepressants.

Dyspepsia is diagnosed by exclusion, i.e., when a subject complains of gastroenteric pain or discomfort (epigastralgia or epigastric discomfort, heavy stomach, heartburn, epigastric fullness, extreme sensation of fullness after a small amount of food, nausea, vomiting, etc.) for a long time, but no structural abnormalities were found even after being examined with endoscopy, abdominal ultrasonography, etc. Dyspepsia is generally diagnosed on the basis of the symptoms reported by the subject, and this makes the diagnosis of dyspepsia difficult. Furthermore, the difficulty of such diagnosis imposes mentally and monetary burdens on the patient and delays appropriate treatment, causing deterioration in the patient's quality of life (QOL).

Therefore, development of an easy and highly accurate method of diagnosing dyspepsia will reduce the mentally and monetary burdens on dyspepsia patients and greatly contribute to its treatment.

One of the primary causes of dyspepsia is a lowering of the gastric-emptying function, and therefore a technique for measuring the gastric-emptying function can be utilized in its diagnosis. However, conventional methods for measuring the gastric-emptying function have the following drawbacks. They are expensive or invasive, the subject is constrained for a long time so that they impose a psychological and physical burden on the subject, and the measurement accuracy is insufficient. For example, among conventional methods for measuring the gastric-emptying function, isotope methods (e.g., scintigraphy or the like) use a radioactive isotope; as a result, the administration of such a method is complicated. Furthermore, since such a method requires an expensive γ-ray camera, its use is restricted to special facilities. In the case of an X-ray impermeable marker method, the marker is not excreted from the stomach simultaneously with the food contents, but is instead excreted from the stomach after all of the food contents have been excreted; as a result, the actual gastric-emptying function cannot be accurately examined. In the case of an acetaminophen method, there is a danger of drug allergy and liver damage due to the side effects of acetaminophen; furthermore, since this drug is subject to other effects in the body, such as absorption in the small intestine, metabolization by the liver, excretion from the kidneys and the like, the gastric-emptying function cannot be accurately examined. Since the concentration of acetaminophen in the blood is measured after administering acetaminophen, the invasive procedure of blood collection is required.

Furthermore, methods such as measuring the endogastric volume and saburra by means of ultrasonic waves (an ultrasound method), measuring the gastric-emptying function by MRI (a magnetic resonance imaging method), evaluating the gastric motor function by measuring with an electrogastrogram (elctrogastrography) and the like have also been proposed for measuring the gastric-emptying function; however, such methods pose the following problems: (i) There are problems in the accuracy of the diagnostic method, (ii) there are no fixed criteria, so that evaluations vary according to the evaluator, and (iii) the subject must be constrained for a long period of time while the diagnosis is being made. (Non-Patent Documents 3 and 4).

WO 97/35622 A1 described a biscuit that is adapted to be ingested by a patient to enable measurement of gastric emptying. To the biscuit dough was added prior to baking Spirulina Platensis alga grown in a ¹³CO₂ atmosphere. These single cell organisms are said to be oxidized after digestion in the small intestine to produce a detectable rise in level of ¹³CO₂ in the patient's breath which is sampled and plotted to enable diagnosis of abnormal gastric emptying rates.

Mattison et. al (CLIN. CANCER. RES. 12, 549-555, 2006) assessed an uracil breath test in the assessment of dihydropyrimidine dehydrogenase activity, and in particular the pharmacokinetic relationship between expired¹³CO₂ and plasma [2-¹³C]dihydrouracil. Also, Inada et. al. (DRUG METAB. DISPOS. 33, 381-387, 2005) studied the relationships among plasma [2-¹³C]uracil concentrations, breath ¹³CO₂-expiration, and DPD activity in the liver in normal and DPD-deficient dogs. This study was designed to examine the effects of a DPD inhibitor on blood concentrations of [2-¹³C]uracil ([¹³C]uracil) and ¹³CO₂ concentration (Delta¹³C) expired in breath after oral or intravenous administration of [¹³C]uracil to DPD-suppressed dogs prepared by pretreatment with 5-(trans-2-bromovinyl)uracil (BVU), a DPD inhibitor.

WO 02/072153 described a method for measuring and evaluating the pyrimidine metabilizability of individual subjects on various fluorouracil-type chemicals typified by 5-fluorouracil which is decomposed in the pyrimidine metabolism pathway; and preparations which are useful in the measurement and evaluation. The pyrimidine metabolizability in vivo can be evaluated by administering a preparation for measuring pyrimidine metabolizability containing a pyrimidine compound or a pyrimidine metabolite which is radio-labeled in at least one of carbon, oxygen and nitrogen atoms serving as the substrate in pyrimidine metabolic enzymes as the active ingredient, and then examining the metabolic behaviors based on the excretion.

WO 2007 013409 aimed at providing an oral preparation which is usable in diagnosing the presence or absence of an abnormality in the capacity to metabolize pyridine, a pyrimidine metabolic speed and so on at a high accuracy with little difference from individual to individual. An isotope-labeled compound and/or a pyrimidine metabolite compound (a) are mixed with a sugar and/or a sugar alcohol (b) and milled. By using the powdery material thus obtained, an oral preparation is produced.
Non-Patent Document 1: Talley Nj et al., Gut 45 (Suppl 2): II 37-42, 1999
Non-Patent Document 2: Quarteo AO et al., Dig Dis Sci 43: 2028-2033, 1998
Non-Patent Document 3: J. Smooth Muscle Res. (Jpn. Sec.) 6: J-75-J-91, 2002
Non-Patent Document 4: J. Smooth Muscle Res. (Jpn. Sec.) 6: J-129-J-138, 2002

### DISCLOSURE OF THE INVENTION

An object of the present invention is to provide a composition for use in a method for measuring the gastric-emptying function used to effectively diagnose dyspepsia. Specifically, the present invention aims to provide a composition that allows an easy and noninvasive measurement of the gastric-emptying function using expired air. The present invention also aims to provide a method for measuring the gastric-emptying function that can be used to effectively diagnose dyspepsia. Specifically, the main objects of the present invention are to provide a composition for use in a method for diagnosing dyspepsia, and a method for diagnosing dyspepsia.

Furthermore, the present invention aims to provide a method for measuring therapeutic effects (including pharmacotherapy) on patients, such as dyspepsia patients, suffering from gastrointestinal disorders caused by an insufficient gastric-emptying function. In particular, the present invention aims to provide a method for measuring the pharmaceutical effects on the patients of medicines affecting the function of the alimentary tract and/or therapeutic effects thereof.

### Means for Solving the Problem

Shown below are the four characteristics desirable for inspection probes for use in diagnosing and evaluating gastric-emptying functions using expired air.
(1) The inspection probe is not absorbed from the stomach, but from the alimentary tracts including and below the duodenum (such as the duodenum, jejunum, ileum, etc.);
(2) Its absorption is not affected by the change in pH (the pH in the alimentary tract);
(3) The probe exhibits high absorption and metabolic rates, and a high excretion rate in expired air as isotope-labeled CO₂ (a high recovery rate); and
(4) The probe is promptly metabolized after absorption.

In order to solve the above problems, the present inventors conducted an intensive search to develop a gastric-emptying function inspection probe having the above characteristics, and found that pyrimidine compounds selected from uracil and thymine labeled with an isotope of C or O have the four characteristics. The present inventors also found that by measuring the amount or behavior of the isotope-labeled CO2 excreted in expired air after orally administering these compounds to a subject, the gastric-emptying function of the subject could be easily measured. They confirmed that the measuring method is effective for diagnosing dyspepsia in a noninvasive manner. They also confirmed that by using the method for measuring the gastric-emptying function using a pyrimidine compound selected from uracil and thymine, the effects of the medicine or like treatment on the subject can be measured, allowing to select a treatment method most desirable and effective for the subject (the patient). The present invention has been accomplished based on such findings.

### Effect of the Invention

The composition of the present invention makes it possible to measure the gastric-emptying function in humans or animals in a simple and accurate manner. Specifically, the composition of the present invention is usable in objectively measuring the gastric motor function, diagnosing the diseases caused by the gastric motor dysfunction, and measuring the pharmaceutical effect of medicines affecting the alimentary tract motor function and/or therapeutic effect on a patient.

The composition of the present invention makes it possible to diagnose dyspepsia by readily and accurately measuring the reduction of a subject's gastric-emptying function. In particular, the diagnostic agent of the present invention uses, as an active ingredient, a labeled piperidine compound selected from uracil and thymine excreted in expired air in a form of a labeled carbon dioxide gas. This allows using the composition by merely testing the expired air, without imposing a psychological or physical burden on the subject. The composition is particularly effective in measuring the effects of treating a dyspeptic patient (including pharmacotherapy), in particular measuring the pharmaceutical effects of medicines affecting the motor function of the alimentary tract or the therapeutic effects thereof.

### BEST MODE FOR CARRYING OUT THE INVENTION

### (I) Composition for use in a method for measuring gastric-emptying function, and (II) for use in a method for diagnosing dyspepsia

The composition of the present invention comprises as an active ingredient a pyrimidine compound that is labeled with at least one of isotopes of C and O, and that is excreted in expired air after being converted into a labeled CO₂ gas in the body.

The compound selected from uracil and thymine used in the composition has a pyrimidine skeleton labeled with at least one of isotopes of C and O, so that it is excreted in expired air after being converted into a labeled CO₂ gas in the body when orally administered.

Preferable properties of the pyrimidine compound selected from uracil and thymine used in the present invention are as follows:
(1) All or almost all thereof is absorbed from the alimentary tracts including and below the duodenum (such as the duodenum, jejunum, ileum) without completely or barely being absorbed in the stomach, and then excreted in expired air as an isotope-labeled CO₂ gas after being decomposed or metabolized.
(2) Its absorption is not readily affected by the change in pH in the alimentary tract. More preferable properties are: (3) Having a high absorption rate, a high metabolic rate, and a high excretion rate (recovery rate) as a labeled CO₂ gas in expired air. A particularly preferable property, in addition to the above properties, is the ability to (4) be promptly metabolized after absorption. Examples of pyrimidine compounds having such properties include uracil and thymine.

The isotopes used in labeling the carbon or oxygen atom in a pyrimidine compound selected from uracil and thymine, is selected from the group consisting of ¹³C, ¹⁴C and ¹⁸O. In particular, ¹³C is especially desirable for use as such an isotope.

Specifically, the pyrimidine compound selected from uracil and thymine selected from uracil and thymine used in the present invention is isotope labeled in such a manner that at least a portion of the CO₂ formed through the pyrimidine metabolic pathway is isotope labeled. Examples of such pyrimidine compounds include 2-¹³C labeled uracil and 2-¹³C labeled thymine

There are no particular restrictions on the method for labeling a pyrimidine compound selected from uracil and thymine with these isotopes, and wide various commonly used methods may be employed. (see Sasaki, "5.1 Use of Stable Isotopes in Clinical Diagnoses"; Kagaku no Ryoiki 107, "Use of Stable Isotopes in Medicine, Pharmacy and Biology", pp. 149-163 (1975) Nankodo; Kajiwara, RADIOISOTOPES, 41, 45-48 (1992); and the like). Furthermore, some isotope-labeled pyrimidine compounds selected from uracil and thymine, such as 2-¹³C labeled uracil, are commercially available and these commercial products are conveniently usable.

There is no particular limitation to the composition of the present invention in terms of its form, components other than the isotope-labeled pyrimidine compound selected from uracil and thymine, proportion of each component, and preparation method of the composition, as long as the pyrimidine compound selected from uracil and thymine contained therein is absorbed in and below the duodenum after oral administration, and excreted in expired air as a labeled CO₂ gas after being metabolized.

There is no limitation to the form of the composition, as long as it can be taken orally. Examples of the forms of the composition include any orally administrable forms, such as solutions (including syrup), suspension, emulsions and like liquids; tablets (with and without coating), chewable tablets, capsules, pills, pulvis (powders), fine particles, granules and like solids.

The application of the composition of the present invention is not limited to formulation, such as pharmaceutical preparation, as long as it contains the labeled pyrimidine compound selected from uracil and thymine and does not adversely affect the effects of the present invention. The labeled pyrimidine compound may be combined with any foodstuff and formed into solid food, fluid food or liquid food.

The composition of the present invention may substantially consist of the isotope-labeled pyrimidine compound selected from uracil and thymine, which is an active ingredient; however, as long as the effects of the present invention are not adversely affected, any pharmaceutically acceptable carriers and/or additives that are generally used in this field may be added.

In this case, there is no limitation to the amount of the isotope-labeled pyrimidine compound selected from uracil and thymine added as an active ingredient. For example, the amount of the isotope-labeled pyrimidine compound selected from uracil and thymine falls in the range from 1 to 95 wt.% per 100 wt.% of the composition, and is preferably controlled within this range.

When the composition of the present invention is formed into, for example, tablets, chewable tablets, capsules, pills, pulvis (powders), fine particles, granules and like solid forms, various carriers and/or additives suitable to such forms may be added.

Specific examples of usable carriers or additives include lactose, sucrose, dextrin, mannitol, xylitol, sorbitol, erythritol, calcium dihydrogen phosphate, sodium chloride, glucose, urine, starch, calcium carbonate, kaolin, crystalline cellulose, silicic acid and like excipients; water, ethanol, simple syrup, glucose liquid, starch liquid, gelatin liquid, carboxymethyl cellulose, sodium carboxymethyl cellulose, shellac, methyl cellulose, hydroxypropylmethyl cellulose, hydroxypropyl cellulose, potassium phosphate, polyvinyl alcohol, polyvinyl pyrrolidone, dextrin, pullulan and like binders; dry starch, sodium alginate, agar powder, laminaran powder, sodium bicarbonate, calcium carbonate, polyoxyethylenesorbitan fatty acid esters, sodium lauryl sulfate, monoglyceride stearate, starch, lactose, carmellose calcium, low substituted hydroxypropyl cellulose, carmellose, croscarmellose sodium, sodium carboxymethyl starch, crospovidone and like disintegrators; saccharose, stearic acid, cacao butter, hydrogenated oil and like disintegration inhibitors; polysorbate 80, quaternary-ammonium salt, laurylsodium sulfate and like absorbefacients; glycerin, starch and like humectants; starch, lactose, kaolin, bentonite, colloidal silicic acid and like adsorbents; purified talc, stearate, boric acid powder, polyethylene glycol, colloidal silicic acid, sucrose fatty acids, hardened oil and like lubricants; citric acid, anhydrous citric acid, sodium citrate, sodium citrate dihydrate, anhydrous sodium monohydrogenphosphate, anhydrous sodium dihydrogenphosphate, sodium hydrogenphosphate and like pH adjustors; iron oxide, β carotene, titanium oxide, edible pigment, copper chlorophyll, riboflavin and like coloring agents; and ascorbic acid, sodium chloride, various sweeteners and like corrigents.

Tablets may be provided with an ordinary coating, if necessary. Specific examples thereof include sugar-coated tablets, gelatin-coated tablets, film-coated tablets, double-coated tablets, multi-coated tablets, etc. Capsules are prepared in a commonly employed method, i.e., mixing the isotope-labeled pyrimidine compound, which is an active ingredient, with.various carriers mentioned above and placing it in a hard gelatin capsule, a soft capsule, etc.

A preferable composition of the present invention for measuring the gastric-emptying function in an accurate manner with little variation attributable to individual subject differences can be obtained by mixing (a) an isotope-labeled pyrimidine compound selected from uracil and thymine and (b) sugar and/or sugar alcohol, and then pulverizing the resulting mixture. The thus-obtained powder is formed into a preparation.

There is no limitation to the sugar and sugar alcohol used here as far as it is pharmaceutically acceptable. Usable sugars include glucose, galactose, fructose, xylose, arabinose, mannose and like monosaccharides; maltose, isomaltose, cellobiose, lactose, sucrose, trehalose and like disaccharides. Among these, glucose and sucrose are preferable. Examples of sugar alcohols include erythritol, mannitol, xylitol, sorbitol, maltitol, hydrogenated palatinose, lactitol, etc. Among these, mannitol, xylitol and erythritol are preferable, and mannitol is particularly preferable. Sugar alcohol is more preferably than sugar used as component (b).

The content of component (a) in the composition is preferably 5 to 20 wt.%, more preferably 6 to 18 wt.%, and particularly preferably 8 to 15 wt.%, per total weight of the composition. The content of component (b) is generally 80 to 95 wt.%, preferably 82 to 94 wt.%, and more preferably 85 to 92 wt.%, per total weight of the composition.

There is no limitation to the ratio of the component (a) to the component (b), and, for example, component (b) may generally be added in 400 to 1,900 parts by weight, preferably 450 to 1,550 parts by weight, and more preferably 550 to 1,150 parts by weight, per 100 parts by weight of component (a).

Such a composition is preferably prepared using a powder material containing component (a) and component (b), and forming the material into preparations. The powder material can preferably be prepared by mixing component (a) with component (b) in such a manner so as to achieve the above-mentioned proportions, and then pulverizing the mixture. There is no limitation to the particle diameter of the powder material, but in order to minimize the variations attributable to individual subject differences and increase the accuracy of the measurement in the gastric-emptying function, the particle diameter at 50% is generally not greater than 40 µm, preferably not greater than 30 µm, and particularly preferably from 5 to 20 µm. The distribution of the particle size is preferably such that the particle diameter at 50% is not greater than 40 µm, and the particle diameter at 90% is not greater than 200 µm; more preferably, the particle diameter at 50% is not greater than 30 µm, and the particle diameter at 90% is not greater than 100 µm; and particularly preferably, the particle diameter at 50% is from 5 to 20 µm, and the particle diameter at 90% is from 10 to 70 µm. Such particle size distribution can be measured by a dry laser method (measurement conditions: focal distance: 100 mm; a number of averaging processes: 10 times; an averaging interval: 5 milliseconds; air pressure: 0.4 MPa).

There is no limitation to the pulverization treatment employed in the preparation of the powder material, but a pulverization treatment using a dry pulverizer is preferable. Examples of usable dry pulverizers include hammer mills, pin mills, jet mills, etc.

The composition may consist of components (a) and (b), and may contain other components as long as the above-mentioned proportions of components (a) and (b) are included. In this case, usable components other than components (a) and (b) include the above-described pharmaceutically acceptable carriers and/or additives (for example, excipients, binders, pH adjustors, disintegrators, absorbefacients, lubricants, coloring agents, corrigents, flavor, etc.). Preferably, those carriers and/or additives had been subjected to the same pulverization treatment as components (a) and (b). There is no limitation to the form of the preparation as long as it can be taken orally, and it may be in the form of a fine particle, granule, pulvis (powder), tablet (with and without coating), capsule, pill, etc. Among these, fine particle, granule and like particles, in particular particles obtained by extrusion granulation, are preferable.

When formed into particles, the average particle diameter of the preparation is, for example, generally not greater than 1,400 µm, preferably within the range of 50 to 1,200 µm, and more preferably within the range of 100 to 1,000 µm. By forming the preparation into particles having such a particle diameter, the gastric-emptying function can be measured in a more accurate manner. The measurement of particle diameters can be conducted by a vibration sieving method (specifically, using a measurement apparatus: Robot Sifter RPS-95, Seishin Enterprise Co., Ltd.; vibration level: 5; shift time: 5 minutes; pulse interval: 1 second).

The amount of the isotope-labeled pyrimidine compound selected from uracil and thymine (active ingredient) contained in a unit dosage of the composition of the present invention cannot be generalized because it varies depending on the types of the measurement sample, the active ingredients etc., and is therefore suitably selected according to each case. For example, when 2-¹³C labeled uracil and like isotope-labeled uracils are used as an isotope-labeled pyrimidine compound, i.e., the active ingredient, generally 1 to 1,000 mg/body and preferably 10 to 100 mg/body of isotope-labeled uracil is contained in one unit dosage of the composition.

The composition for use in a method for measuring the gastric-emptying function of the present invention allows measuring the gastric-emptying function of a subject when taken orally, by measuring the amount or behavior of the labeled CO2 gas excreted in expired air.

Specifically, the composition of the present invention, after being taken orally, enters into the stomach, and is then excreted from the pylorus of the stomach by systolic and diastolic motion and/or peristaltic movement. After being excreted from the pylorus of the stomach, the isotope-labeled pyrimidine compound selected from uracil and thymine, which is an active ingredient, is promptly absorbed and metabolized in the alimentary tract including and below the duodenum (the duodenum, jejunum, ileum, etc.), and then excreted in expired air as a labeled CO₂ gas. One of the major features of the isotope-labeled pyrimidine compound selected from uracil and thymine used in the present invention is that it is not absorbed, or barely absorbed, in the stomach, and that it is promptly absorbed and metabolized after being discharged from the stomach, and finally excreted in expired air as a labeled CO₂ gas. Therefore, the behavior of the labeled CO₂ gas excreted in expired air (specifically, for example, the behavior is expressed as the proportion of the isotope-labeled CO₂ gas in the ¹²CO₂ excreted in expired air, (isotope-labeled CO₂/¹²CO₂)), depends on the gastric emptying rate (gastric emptying time) of the composition of the present invention, i.e., the isotope-labeled pyrimidine compound selected from uracil and thymine.

The following factors can be used as the indices expressing the gastric-emptying function. Specifically, the amount of the ¹³CO₂ gas in expired air after a predetermined time from the administration of the composition, the carbon dioxide gas value Δ(‰) (the difference of the ¹³CO₂/¹²CO₂ concentration ratios (δ¹³C values) before and after the administration of the composition in expired air collected), or the initial speed of the ¹³CO₂ gas. For example, when the carbon dioxide gas value Δ(‰) or the initial speed of the ¹³CO₂ gas of a healthy subject is determined as a criterion value, and if the subject exhibited a carbon dioxide gas value Δ(‰) or the initial speed lower than that of a healthy subject, the subject is diagnosed as having a reduced gastric-emptying function.

The composition of the present invention may be taken singly, together with an experimental diet, or immediately before or after taking the experimental diet. One preferable example of the method is that the composition of the present invention is taken immediately after taking an experimental diet. There is no limitation to the experimental diet taken, as far as it does not adversely affect the measurement of the gastric-emptying function using the composition of the present invention, and it may be a solid food, fluid food or liquid food.

As described above, one of the main causes of dyspepsia (non-ulcer dyspepsia symptoms in the alimentary tract) is dysfunction of the alimentary tract motor, especially lowering of the gastric-emptying function. Accordingly, the composition for use in a method for measuring the gastric-emptying function of the present invention is effectively usable as an agent for diagnosing dyspepsia, especially dyspepsia mainly caused by insufficiencies in the gastric-emptying function (for example, dysmotility-type dyspepsia). Therefore, all explanations relating to the composition for use in a method for measuring the gastric-emptying function of the present invention can also be applied to the composition for use in a method for diagnosing dyspepsia.

### (III) Method for measuring gastric-emptying function and (iv) Method for diagnosing dyspepsia

The present invention also relates to a method for measuring the gastric-emptying function conducted using the composition described above. The measurement of the gastric-emptying function can be conducted by orally administering the composition of the present invention, to animals or humans; collecting expired air; and measuring the amount or behavior of the labeled CO₂ gas excreted in expired air.

When ¹³C is used as an isotope, the gastric-emptying function can be measured according to an ordinary ¹³C breath test, i.e., after orally administering the composition of the present invention to a subject, expired air is collected over the lapse of time, and, with the amount of ¹³CO₂ excreted in expired air being expressed as ¹³CO₂/¹²CO₂ ratio (δ¹³C value), the behavior is observed over time.

The composition for use in a method for measuring the gastric-emptying function of the present invention contains, as an active ingredient, an isotope-labeled pyrimidine compound selected from uracil and thymine that is not absorbed or is barely absorbed in the stomach. After being excreted from the stomach, the isotope-labeled pyrimidine compound selected from uracil and thymine is hardly affected by the pH in the alimentary tract, and is promptly absorbed and metabolized in and below the duodenum (the duodenum, jejunum, ileum, etc.). Thereafter, a large percentage thereof is excreted into expired air as an isotope-labeled CO₂ gas. This achieves the measurement directly and accurately reflecting the gastric excretion motor function. By preparing the composition for measuring the gastric-emptying function using a powder material containing an isotope-labeled pyrimidine compound selected from uracil and thymine and a sugar and/or sugar alcohol, the variance between subjects can be reduced and the gastric excretion motor function can be accurately measured.

The measurement of the gastric-emptying function is conducted with higher accuracy by administering the composition of the present invention not only once but several times in a repeated manner and under various conditions including after fasting, after eating, etc. The measurement and analysis methods of the isotope-labeled CO₂ in expired air vary depending on whether the isotope used is radioactive or non-radioactive, but generally used methods include the liquid scintillation counter method, mass spectrometry, infrared spectroscopy, emission spectrometry, and the magnetic resonance spectrum method. From the viewpoint of measurement accuracy, infrared spectroscopy and mass spectrometry are preferable.

The method for administering the composition for use in a method for measuring the gastric-emptying function of the present invention may be the same as that described above, but is not limited to this.

The amount of the isotope-labeled pyrimidine compound selected from uracil and thymine per unit dose of the composition of the present invention cannot be generalized because it varies depending on the types of the isotope-labeled compound, etc., and may be suitably selected in each case. For example, when measurement is conducted by an expired air test using 2-¹³C uracil as an isotope-labeled pyrimidine compound, preferably 1 to 2,000 mg, and more preferably 10 to 300 mg of 2-¹³C uracil is contained in a preparation per unit dose.

By employing the method for measuring the gastric-emptying function of the present invention, the drop or acceleration of a subject's excretion function can be diagnosed and measured. Specifically, the diagnosis can be conducted by the comparison between the amount or behavior of the isotope-labeled CO₂ gas excreted in expired air from a subject measured by the above method with the standard control (the amount or behavior of the isotope-labeled CO₂ gas excreted in expired air from a healthy subject).

The gastric-emptying function of a subject can be diagnosed and measured, for example, in the manner as describe below.

After administering the composition of the present invention to a subject, the amounts of the isotope-labeled carbon dioxide (¹³CO₂) excreted in expired air or carbon dioxide gas value Δ(‰) (the difference between the δ¹³C values before and after the administration of the composition) were measured with lapse of time, and compared its excretion pattern with that of the standard control (from a healthy subject).

Lowering of the gastric-emptying function can also be detected from the initial speed of the ¹³CO₂ excreted in expired air. This method further shortens the total hours the subject has to be constrained. In this case, if the initial speed of the ¹³CO₂ excreted into expired air is slower than that of the standard control (the healthy subject), the gastric-emptying function is judged to be reduced.

As described above, one of major causes of dyspepsia (non-ulcerative upper-gastrointestinal tract syndrome) is alimentary tract motor dysfunction, in particular the lowering of the gastric-emptying function. Therefore, the above-explained method for measuring the gastric-emptying function can also be effectively employed for diagnosing dyspepsia, in particular dyspepsia mainly attributable to insufficiencies in the gastric-emptying function (for example, dysmotility-type dyspepsia). Accordingly, all explanations of the method for measuring the gastric-emptying function of the present invention can be applied to a dyspepsia diagnosis method.

### (V) Method for measuring pharmaceutical or therapeutic effect

By employing the method for measuring the gastric-emptying function described above, the pharmaceutical effect of the gastrointestinal medicine, in particular, a medicine that affects the alimentary tract motor function, or therapeutic effect on each subject can be measured. Specifically, the measurement can be conducted by comparing the gastric-emptying functions measured using the composition for use in a method for measuring the gastric-emptying function of 5 the present invention before and after administering to a subject a gastrointestinal medicine, in particular, a medicine relating to the alimentary tract motor function. This makes it possible to evaluate the pharmaceutical effect of the medicine itself. Furthermore, the therapeutic effect of the medicine on each subject can also be measured. As a result, this method can be used as a means to suitably select a medicine applicable to each subject.

Examples of the medicines affecting the alimentary tract motor function include alimentary tract motor function improving agents, alimentary tract motor function accelerators, and alimentary tract motor function activation agents (specifically, acetylcholine agonists, dopamine receptor antagonists, dopamine D₂ receptor antagonists, serotonin receptor agonists, opioid receptor agonist, Chinese medicines (Rikkunshi-to, Hange-shashin-to, and Annaka powder); alimentary tract motor function inhibitors (anticholinergic agents, muscarinic receptor antagonists, etc.) and the like, which control the peristaltic movement of the stomach in an accelerating or inhibitory manner.

In this method, the subject may be a patient with dyspepsia, especially a patient whose dyspepsia is mainly caused by a gastric motor dysfunction (a dysmotility-type dyspepsia patient). In this case, the pharmacotherapy effect on each dyspepsia patient can be measured, so that a suitable medicine, e.g., a medicine affecting the alimentary tract motor functions (alimentary tract motor function improving agents, alimentary tract motor function accelerators, or alimentary tract motor function activation agents) can be selected. In other words, the method is effectively usable in measuring the pharmaceutical effect of a medicine for a dyspepsia patient, in particular, a medicine affecting the alimentary tract motor function, or measuring the therapeutic effect of a medicine on a dyspepsia patient. The above explanation of the method for measuring the gastric-emptying function can also be applied to the specific measuring method of the present invention.

### EXAMPLES

The present invention is further explained with reference to Examples and Experimental Examples. However, the scope of the present invention is not limited to these Examples, etc.

### Example 1 Solution

2-¹³C uracil (molecular weight: 113.08; Cambridge Isotope Laboratory; 100 mg) was dissolved in 50 ml of 0.1N-NaOH/saline solution (adjusted), preparing a composition taking a form of an aqueous solution (containing 2-¹³C uracil at a concentration of 20 µmol/ml).

### Example 2 Granules

### (1) Preparation of granules

2-¹³C uracil (Cambridge Isotope Laboratory, 20 g) and D-mannitol (Mannite; Kyowa Hakko Kogyo Co., Ltd., 380 g) were mixed, introduced into a SampleMill (KIIWG-1F; Fuji Paudal Co., Ltd.) and then subjected to pulverization while mixing (pulverization conditions; the number of revolutions of the pulverization rotor: 12,800 rpm; the number of revolutions of the sample supply motor: about 10 rpm; screen: 1 mm punch screen), thereby preparing a powder material. The thus-obtained powder material (200 g) was weighed, placed in a speed kneader (NSK-150; Okada Seiko Co., Ltd.), and then kneaded after adding 20 g of purified water. Subsequently, the thus-obtained wet powder was extruded using an extrusion granulator equipped with a dome-shaped die having openings of φ1 mm (Dome Gran DG-1L; Fuji Paudal Co., Ltd.), and then dried at 60°C using a ventilation drier (SPHH-200; ESPEC Corp.). From the dried preparation, after passing through a sieve having openings of 1,400 µm but not through openings of 355 µm, 5 wt.% of 2-¹³C uracil-containing granules were obtained.

The particle diameters of the thus-obtained 5 wt.% of 2-¹³C uracil-containing granules were measured by vibration sieving (specifically, measurement apparatus: Robot Sifter RPS-95, Seishin Enterprise Co., Ltd.; vibration level: 5; shift time: 5 minutes; interpulse interval: 1 second). Table 1 shows the results.

**Table 1**

| Particle Diameter | Content (wt.%) |
|---|---|
| Not less than 1400 µm | 2.09 |
| 1000 µm to less than 1400 µm | 7.29 |
| 850 µm to less than 1000 µm | 22.07 |
| 710 µm to less than 850 µm | 59.04 |
| 500 µm to less than 710 µm | 8.99 |
| 355 µm to less than 500 µm | 0.09 |
| 250 µm to less than 355 µm | 0.00 |
| 150 µm to less than 250 µm | 0.09 |
| Less than 150 µm | 0.34 |
| Total | 100.0 |

### (2) Evaluation of solubility

Tap water (100 mL) was placed in a 200 mL beaker at room temperature, 2,000 mg of the above-obtained granules were added thereto while stirring at 200 rpm using a magnetic stirrer (RCN-7D; EYELA), and then the time until dissolution was measured by visual observation. When three minutes had passed after the addition of the granules, the remaining, undissolved preparation was observed by visual observation. The results show that the time before completion of the dissolution was 1 minute and 10 seconds, and that very little preparation remained after 3 minutes.

### Example 3 Granules

2-¹³C uracil (Cambridge Isotope Laboratory, 20 g) and D-mannitol (Mannite; Kyowa Hakko Kogyo Co., Ltd.; 180 g) were mixed, introduced into a SampleMill (KIIWG-1F; Fuji Paudal Co., Ltd.) and then subjected to pulverization while mixing (pulverization conditions: the number of revolutions of the pulverization rotor: 12,800 rpm; the number of revolutions of the sample supply motor: about 10 rpm; screen: 1 mm punch screen), thereby preparing a powder material. The thus-obtained powder material (144 g) was weighed, placed in a speed kneader (NSK-150; Okada Seiko Co., Ltd.), and then kneaded after adding 14.4 g of purified water. Subsequently, the thus-obtained wet powder was extruded using an extrusion granulator equipped with a dome-shaped die having openings of φ1 mm (Dome Gran DG-1L; Fuji Paudal Co., Ltd.), and then dried at 60°C using a ventilation drier (SPHH-201, ESPEC Corp.). From the dried preparation, after passing through a sieve having openings of 1,400 µm but not through openings of 355 µm, 10 wt.% of 2-¹³C uracil-containing granules were obtained.

### Example 4 Tablets

2-¹³C uracil (Cambridge Isotope Laboratory, 100 g) and lactose (H.M.S, 60 g), corn starch (Cornstarch; Nihon Shokuhin Kako Co., Ltd.; 25 g), crystalline cellulose (CEOLUS PH301; Asahi Kasei Corporation; 10 g) and hydroxypropyl cellulose (HPC-L fine powder; Nippon Soda Co., Ltd.; 4 g) were placed in a speed kneader (NSK-150; Okada Seiko Co.; Lid.) and mixed, and then kneaded after adding 40 g of purified water. Subsequently, the thus-obtained kneaded powder was granulated using a speed mill equipped with a 3 mm punch screen (ND-02; Okada Seiko Co., Ltd.), and then dried by a ventilation drier (SPHH-200; ESPEC Corp.) having a temperature of 70°C. The dried granules were subjected to sizing by passing through a No. 16 sieve. Magnesium stearate (Taihei Chemicals Limited, 1 g) was added to 199 g of the granules after sizing, giving granules for tablets. The resulting granules for tablets were formed into tablets each having a weight of 200 mg using a single-shot tableting machine equipped with a punch and die having a corner angle R of φ8 mm (No.2B; Kikusui Seisakusho Ltd.).

### Example 5 Powder preparation

### (1) Formation of powder preparation

2-¹³C uracil (Cambridge Isotope Laboratory, 20 g) and D-mannitol (Mannite; Kyowa Hakko Kogyo Co., Ltd.; 180 g) were well mixed, placed in a SampleMill (SAM; Nara Machinery Co., Ltd. and subjected to pulverization while mixing (shape of the grinding vane: pin-type; number of revolutions of rotor: 4,000 rpm; screen: 3 mm punch screen), producing powder preparations.

### (2) Measurement of distribution of particle size

The particle size distribution of the powder preparations was measured using a dry particle size distribution measurement apparatus (LDSA-1500A, Tohnichi Computer Applications Co., Ltd.; focal distance: 100 mm; number of averaging processes: 10 times; averaging interval: 5 milliseconds; and air pressure: 0.4 MPa). Based on the obtained particle size distribution, particle diameter at 10% (10% D (µm)), particle diameter at 50% (50% D (µm)), and particle diameter at 90% (90% D (µm)) were calculated. Table 2 shows the results.

**Table 2**

| 10% D (µm) | 50% D (µm) | 90% D (µm) |
|---|---|---|
| 5.74 | 14.95 | 56.58 |

As shown in Table 2, the resulting particles had very small diameters, and a satisfactory pulverization effect was obtained.

### Examples 6-10 and 11-15

A solution (Example 6), granules (Examples 7 and 8), tablets (Example 9) and powder preparations (Example 10) were prepared in the same manner as in Examples 1 to 5, except that 2-¹³C thymine was used instead of 2-¹³C uracil. A solution (Example 11), granules (Examples 12 and 13), tablets (Example 14) and powder preparations (Example 15) were prepared in the same manner as in Examples 1 to 5, except that 2-¹³C cytosine was used instead of 2-¹³C uracil.

### Experimental Example 1

Rats (female, Wister rat, 8 weeks old) were anesthetized with pentobarbital and subjected to laparotomy. Thereafter, each of the stomach, duodenum, jejunum and ileum was tied to form a loop in each of the digestive organs (e.g., the stomach, duodenum, jejunum, and ileum). Specifically, the stomach was tied at the pylorus portion, the duodenum was tied at the pylorus portion and 20 cm below the pylorus portion, the jejunum was tied 10 cm below the Treitz' ligament and further 20 cm therebelow, and the ileum was tied 20 cm above the cecum and ileocecal portion.

Subsequently, an aqueous solution of 20 µmol/mL 6-¹⁴C uracil (Moravek Biochemicals, Inc.) dissolved in distillation water was injected at 1 mL/kg to each alimentary tract loop using an injection needle, and the injection portion was sealed using an adhesive. Blood was collected 5, 10, 20, 30, 45 and 60 minutes after the injection of the aqueous 6-¹⁴C uracil solution from the jugular vein using an injection syringe, and then transferred into a tube containing a serum separation agent. The thus-collected blood was centrifuged for 15 minutes at 3,000 rpm, obtaining serum. Subsequently, the radioactivity in the serum was measured using a liquid scintillation counter. Table 1 shows the results.

As is clear from the results of Table 1, uracil is not absorbed or is barely absorbed in the stomach, but is absorbed in alimentary tracts including and below the duodenum (e.g., the duodenum, jejunum, and ileum).

### Experimental Example 2

2-¹³C uracil (Cambridge Isotope Laboratory) was dissolved in Britton-Robinson buffer solutions of differing pH values (pH 2, 4, 6 and 8) to prepare a saturated solution. Each solution was subjected to high performance liquid chromatography, the amount of the 2-¹³C uracil dissolved in the solution was measured, and then the solubility (w/v%) was calculated. Table 3 shows the results.

**Table 3**

| pH of buffer solution | Solubility (w/v%) |
|---|---|
| 2.0 | 0.28 |
| 4.0 | 0.28 |
| 6.0 | 0.28 |
| 8.0 | 0.29 |

As is clear from the results, the solubility of uracil is almost unchanged depending on the pH of the solution (i.e., the alimentary tract pH).

### Experimental Example 3

An aqueous solution containing 6-¹⁴C uracil (Moravek Biochemicals, Inc.) was orally administered to rats (female, Wister rats, 8 weeks old: n=3) and dogs (female, beagle, 11 kg: n=3) once in an amount of 20 µmol/kg under fasting. Up to 168 hours after the administration, the amount of the metabolite excreted in expired air, urine and feces were measured with a liquid scintillation counter using the radioactivity as its index, so that the accumulated excretion rate was obtained.

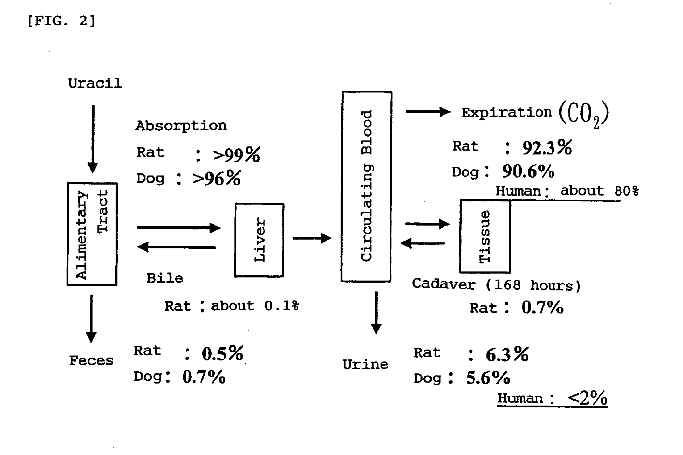

The granules containing 2-¹³C uracil (Cambridge Isotope Laboratory) (100 mg calculated as the amount of 2-¹³C uracil) prepared in Example 2 were orally administered to humans (12 cases), and then the rates of the metabolite excreted into urine and expired air were measured. The excretion rate to urine was expressed as the metabolite excretion rate (the accumulated excretion rate) in the urine pooled for 12 hours from the administration of 2-¹³C uracil, calculated based on the metabolite concentration in urine measured using an LC/MS/MS and the amount of urine. The excretion rate in expired air was measured based on the ¹³CO₂ concentration excreted in expired air (before administration, and 10, 20, 30, 40, 50, 60, 90 minutes and 2, 4, 6, 8, 12 hours after administration) using a GC/MS and the conversion equation of Ghoos, et al. (Ghoos YF, Maes BD, Geypens BJ, et al., Measurement of gastric-emptying function of solids by means of a carbon-labeled octanoic acid breath test. Gastroenterology 1993; 104: 1640-7). FIG. 2 shows the results.

As shown in FIG. 2, the uracil administered to rats, dogs and humans was metabolized within 12 hours from administration, and a large amount, i.e., over 80%, thereof was excreted into expired air as carbon dioxide gas.

### Experimental Example 4

"Propantheline", an anticholinergic agent, was intravenously administered to rats (female, Wister rat, 8 weeks old: n=3) in such an amount of 1 mg/kg to obtain model animals whose gastric-emptying function was decreased (a delayed gastric-emptying model). Five minutes after the administration of propantheline, 10 mg/kg of aqueous 2-¹³C uracil solution was orally administered to each rat, and expired air was collected by suction 5, 10, 20, 30, 40, 50 and 60 minutes after administration at the rate of 100 mL/60 sec, using an expired air collection device for rats. The ¹³CO₂ concentrations in the thus-collected expired air samples and expired air samples (pre) collected in the same manner before administration of 2-¹³C uracil were measured using a GC-MS (ABCA-G; Europe Scientific Ltd.). As a control experiment, 2-¹³C uracil (10 mg/kg) was administered in the same manner as described above to rats to which propantheline was not intravenously injected, and therefore having normal gastric-emptying function. The expired air samples were collected over time, and the ¹³CO₂ concentration in each expired air sample was measured.

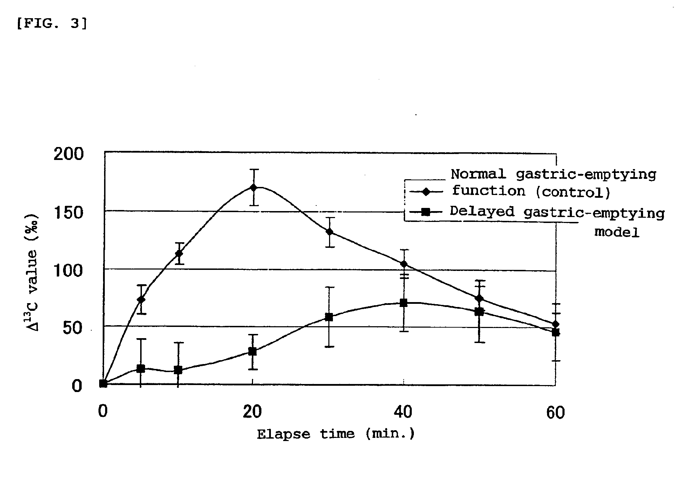

FIG. 3 shows the change in the ¹³CO₂ concentration in expired air after administration of 2-¹³C uracil. In FIG. 3, the vertical axis indicates the Δ¹³C value (‰) which is the difference between the δ¹³C values (‰) (¹³CO₂/¹²CO₂ concentration ratio in expired air) in the expired air samples collected before and after administration of 2-¹³C uracil. The horizontal axis indicates the time (in minutes) collected expired air from the administration of 2-¹³C uracil. As is clear from FIG. 3, the ¹³CO₂ concentration (Δ¹³C value (‰)) excreted in expired air from the rats having reduced gastric-emptying function by administering propantheline was significantly lower than that of normal rats. From this result, it became clear that the gastric-emptying function can be measured based on the change of the ¹³CO₂ concentration in expired air after the administration of 2-¹³C uracil; and that by comparing the change of the ¹³CO₂ concentration in expired air after the administration of 2-¹³C uracil with that of controls having normal gastric-emptying function, drop or acceleration in the gastric-emptying function can be detected.

### Experimental Example 5

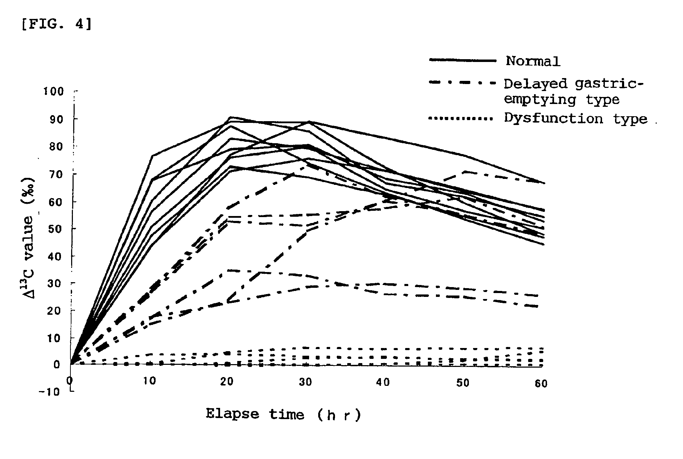

The 2-¹³C uracil granules prepared in Example 2 were orally administered to human patients (on or before 20 days from the extraction of the stomach; a total of 20 cases, with 7 cases undergoing total gastric resection) who were suspected of having post-operative gastroparesis, in a 100 mg amount calculated as the 2-¹³C uracil amount. The expired air was collected 10, 20, 30, 40, 50 and 60 minutes after the administration, and the ¹³CO₂ concentration in each of the expired air samples including expired air samples (pre-) collected before administering 2-¹³C uracil was measured using a GC/MS. Subsequently, the change of the ¹³CO₂ concentration (Δ¹³C(‰)) in expired air was calculated. FIG. 4 shows the results.

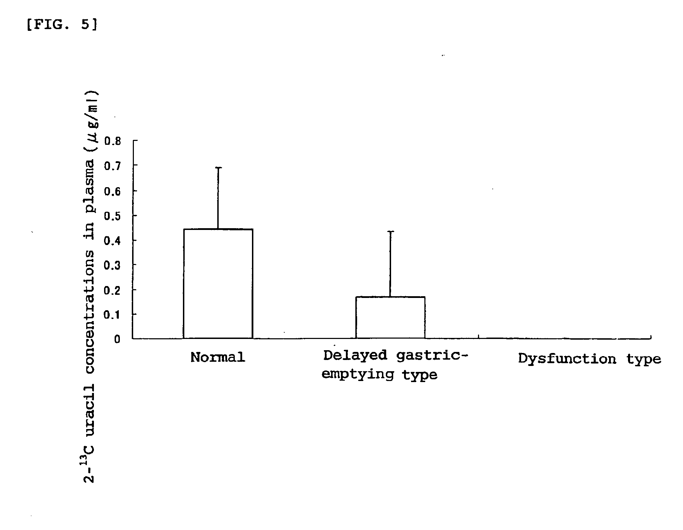

As shown in FIG. 4, based on the expired air test of the present invention using the 2-¹³C uracil, the human patients (20 cases) can be divided into three groups: patients with normal gastric-emptying function (normal type: solid line), patients with decreased gastric-emptying function (delayed gastric-emptying type: dashed line), and patients with insufficient gastric-emptying function (dysfunction type: dotted line). Regarding these patients, 2-¹³C uracil concentrations in plasma were measured 20 minutes after the administration of 2-¹³C uracil. As shown in FIG. 5, patients with decreased.gastric-emptying function (delayed gastric emptying), and patients with insufficient gastric-emptying function exhibited lowered 2-¹³C uracil concentrations in plasma corresponding to the reduction of the gastric-emptying function. This indicates that expired air test of the present invention using 2-¹³C uracil accurately reflects the gastric-emptying function.

### Experimental Example 6 Accuracy of diagnosis evaluation

Granules of Example 2 (1 g each) were orally administered to three healthy subjects (subjects A, B and C); their expired air samples were collected over time, and then the ¹³CO₂ concentration in the expired air was measured using a GC-MS analysis apparatus (ABCA-G; Europe Scientific Ltd.).

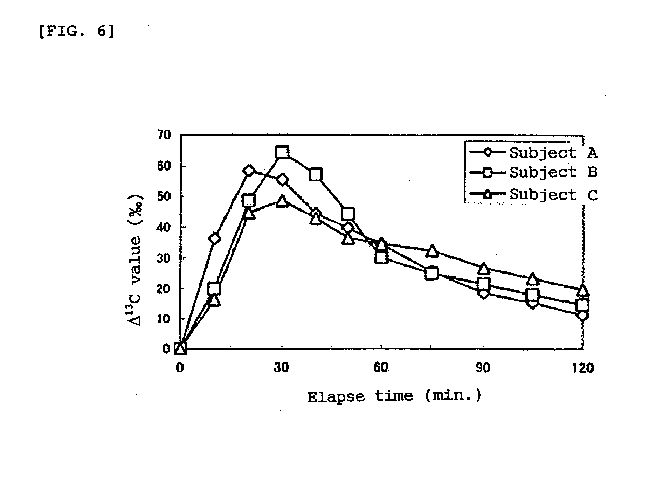

FIG. 6 shows the change in the ¹³CO₂ concentration in expired air after the administration of the preparation. In FIG. 6, the vertical axis indicates the Δ¹³C value (‰), which is the difference between the δ¹³C values (‰) (¹³CO₂/¹²CO₂ concentration ratio in expired air) in the expired air samples collected before and after administration of 2-¹³C uracil. The horizontal axis indicates the time (in minutes) collected expired air from the administration of the granules. As is clear from FIG. 6, when the granules prepared by mixing and pulverizing a powder material containing an isotope-labeled pyrimidine compound and a sugar and/or sugar alcohol as those prepared in Example 2 were used in an expired air test, the individual subjects showed similar patterns of change in ¹³CO₂ concentration and the variance attributable to the differences in the individual subjects can be reduced. This result indicates that by measuring the gastric-emptying function using, as the index, the ¹³CO₂ concentration in expired air 20 to 30 minutes after administering the above-mentioned preparation, dyspepsia can be diagnosed in a quick and accurate manner with little variance between subjects.

### BRIEF DESCRIPTION OF DRAWINGS

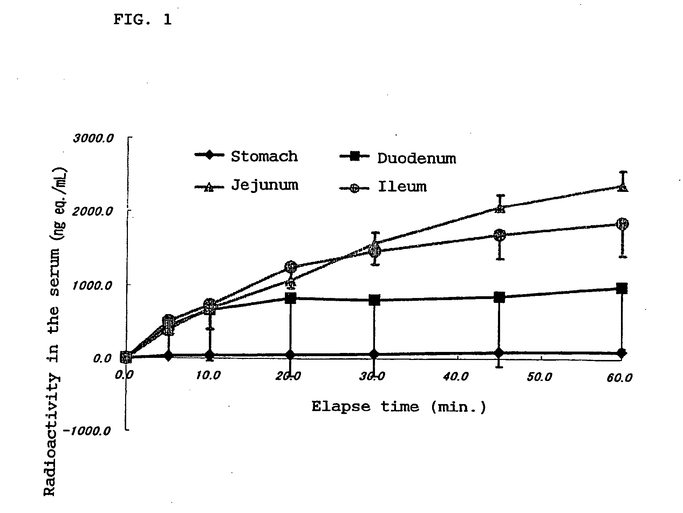

FIG. 1 shows the absorption of uracil (6-¹⁴C uracil) in the stomach, duodenum, jejunum, and ileum.
FIG. 2 shows in the body dynamics of uracil.
FIG. 3 shows the behavior with lapse of time of ¹³CO₂ excreted in expired air after orally administering 2-¹³C uracil to delayed gastric-emptying model rats (pretreatment with propantheline) and rats with normal gastric-emptying function (control) in Experimental Example 3.
FIG. 4 shows the behavior with lapse of time of ¹³CO₂. excreted in expired air when 2-¹³C uracil was administered to 20 patients suspected of having gastroparesis in Experimental Example 4.
FIG. 5 shows the 2-¹³C uracil concentration in plasma 20 minutes after the administration of 2-¹³C uracil in the patients divided into three groups based on the results shown in FIG. 4: i.e., patients with normal gastric-emptying function, patients with decreased gastric-emptying function, and patients with insufficient gastric-emptying function.
FIG. 6 shows the behavior with lapse of time of ¹³CO₂ excreted in expired air when the granules of Example 2 were orally administered to three healthy subjects (subjects A, B and C).

## Claims

1. A composition for use in a method for measuring the gastric-emptying function comprising at least one pyrimidine compound selected from uracil and thymine as an active ingredient,
the pyrimidine compound being labeled with at least one member selected from the group consisting of ¹³C, ¹⁴C and ¹⁸O, converted into labeled CO₂ in the body, and excreted in expired air.

2. The composition according to claim 1, wherein the composition contains any pharmaceutically acceptable carriers and /or additives.

3. The composition according to claim 2, wherein the composition is formed into a solid form selected from the group consisting of tablet, chewable tablet, capsule, pill, powders, fine particles and granules.

4. A composition for use in a method for diagnosing dyspepsia, wherein the composition is consisting of a composition as defined in any one of claims 1 and 3.

5. The composition according to claim 4, wherein the dyspepsia is caused by an insufficient gastric-emptying function.

6. A method for measuring the gastric-emptying function comprising:
using expired air collected from a subject who had orally taken a composition of any one of claims 1 to 3, as a test sample; and
measuring *in vitro* the amount of the labeled CO₂ in the test sample.

7. The method for measuring the gastric-emptying function according to claim 6, which comprises:
using expired air from a subject in whom a drop or acceleration in the gastric-emptying function is suspected and who had orally taken a composition of any one of claims 1 to 3, as a test sample;
measuring *in vitro* the amount of the labeled CO₂ in the test sample; and
comparing the measured value with the amount of the labeled CO₂ in expired air collected from a healthy subject who had taken the same composition.

8. A method for diagnosing dyspepsia comprising:
using expired air collected from a subject who had orally taken the composition of claim 4 or 5, as a test sample;
measuring *in vitro* the amount of the labeled CO₂ in the test sample; and
measuring the gastric-emptying function.

9. The method for diagnosing dyspepsia according to claim 8, which comprises:
using expired air collected from a subject in whom dyspepsia is suspected and who had orally taken the composition of claim 4 or 5, as a test sample;
measuring in vitro the amount of the labeled CO₂ in the test sample;
comparing the measured value with the amount of labeled CO₂ in expired air collected from a healthy subject who had taken the same composition; and
measuring the gastric-emptying function.

10. The method for diagnosing dyspepsia according to claim 8 or 9, wherein the dyspepsia is caused by an insufficient gastric-emptying function.

11. A method for measuring a gastrointestinal treatment effect on a subject comprising:
using, as a test sample, expired air collected from a subject who had orally taken the composition of any one of claims 1 to 5 before and after conducting the gastrointestinal treatment; and
comparing the amounts of the labeled CO₂ excreted in expired air before and after the gastrointestinal treatment.

12. A method for evaluating a pharmaceutical effect of a gastrointestinal medicine or a therapeutic effect on a subject comprising:
using, as a test sample, expired air collected from a subject who had orally taken the composition of of any one claims 1 to 5 before and after administering the gastrointestinal medicine to the subject; and
comparing the amounts of the labeled CO₂ excreted in expired air before and after the administration of the gastrointestinal medicine.

13. The measuring method according to claim 12, wherein the gastrointestinal medicine affects the alimentary tract motor function.

14. The measuring method according to claim 12, which is used to measure the pharmaceutical effect of a gastrointestinal medicine or the therapeutic effect on a patient suffering from dyspepsia caused by an insufficient gastric-emptying function.

15. Use of a pyrimidine compound selected from uracil and thymine for producing a dyspepsia diagnostic agent, the pyrimidine compound being labeled with either or both isotopes of C and O, converted into an isotope-labeled CO₂ gas in the body, and then excreted in expired air.

## Patentansprüche

1. Zusammensetzung zur Verwendung in einem Verfahren zum Messen der Magenentleerungsfunktion, umfassend mindestens eine Pyrimidinverbindung, die ausgewählt ist aus Uracil und Thymin, als Wirkstoff,
worin die Pyrimidinverbindung mit zumindest einem Element markiert ist, das ausgewählt aus der Gruppe bestehend aus ¹³C, ¹⁴C und ¹⁸O, welches im Körper zu markiertem CO₂ umgewandelt wird und in ausgeatmeter Luft ausgeschieden wird.

2. Zusammensetzung gemäß Anspruch 1, worin die Zusammensetzung jegliche pharmazeutisch akzeptable Träger und/oder Additive enthält.

3. Zusammensetzung gemäß Anspruch 2, worin die Zusammensetzung zu einer festen Form verarbeitet ist, ausgewählt aus der Gruppe bestehend aus einer Tablette, einer kaubaren Tablette, einer Kapsel, eine Pille, Pulvern, Feinpartikeln und Granalien.

4. Zusammensetzung zur Verwendung in einem Verfahren zur Diagnose von Verdauungsstörungen, worin die Zusammensetzung aus einer Zusammensetzung, wie sie in irgendeinem der Ansprüche 1 bis 3 definiert ist, besteht.

5. Zusammensetzung gemäß Anspruch 4, worin die Verdauungsstörung durch eine unzureichende Magenentleerungsfunktion verursacht wird.

6. Verfahren zum Messen der Magenentleerungsfunktion, umfassend:
Verwenden von ausgeatmeter Luft, die von einem Subjekt genommen wurde, welches oral eine Zusammensetzung gemäß irgendeinem der Ansprüche 1 bis 3 genommen hat, als Probe; und
Messen der Menge von markiertem CO₂ in der Probe in vitro.

7. Verfahren zum Messen der Magenentleerungsfunktion gemäß Anspruch 6, welches umfasst:
Verwenden von ausgeatmeter Luft von einem Subjekt, bei dem eine Verlangsamung oder Beschleunigung der Magenentleerungsfunktion vermutet wird, und welches oral eine Zusammensetzung gemäß irgendeinem der Ansprüche 1 bis 3 genommen hat, als Probe;
in vitro-Messen der Menge des markierten CO₂ in der Probe; und
Vergleichen des gemessenen Wertes mit der Menge des markierten CO₂ in ausgeatmeter Luft, welche von einem gesunden Subjekt gesammelt wurde, welches die gleiche Zusammensetzung genommen hat.

8. Verfahren zur Diagnose von Verdauungsstörungen, umfassend:
Verwenden von ausgeatmeter Luft, welche von einem Subjekt gesammelt wurde, welches oral die Zusammensetzung gemäß Anspruch 4 oder 5 genommen hat, als Probe;
in vitro-Messen der Menge des markierten CO₂ in der Probe, und
Messen der Magenentleerungsfunktion.

9. Verfahren zur Diagnose von Verdauungsstörungen gemäß Anspruch 8, welches umfasst:
Verwenden von ausgeatmeter Luft, welche von einem Subjekt gesammelt wurde, bei dem Verdauungsstörungen vermutet werden, und welches oral die Zusammensetzung gemäß Anspruch 4 oder 5 genommen hat, als Probe;
in vitro-Messen der Menge des markierten CO₂ in der Probe;
Vergleichen des gemessenen Wertes mit der Menge von markiertem CO₂ in ausgeatmeter Luft, die von einem gesunden Subjekt gesammelt wurde, welches die gleiche Zusammensetzung genommen hat; und
Messen der Magenentleerungsfunktion.

10. Verfahren zur Diagnose von Verdauungsstörungen gemäß Anspruch 8 oder 9, worin die Verdauungsstörung durch eine unzureichende Magenentleerungsfunktion verursacht wird.

11. Verfahren zum Messen der Wirkung einer Verdauungstraktbehandlung auf ein Subjekt, umfassend:
Verwenden von ausgeatmeter Luft, die von einem Subjekt gesammelt wurde, welches oral die Zusammensetzung gemäß irgendeinem der Ansprüche 1 bis 5 genommen hat, als Probe, vor und nach dem Durchführen der Verdauungstraktbehandlung; und
Vergleichen der Mengen von markiertem CO₂, das in die ausgeatmete Luft ausgeschieden wurde, vor und nach der Verdauungstraktbehandlung.

12. Verfahren zum Bewerten einer pharmazeutischen Wirkung einer Verdauungstraktmedizin oder einer therapeutischen Wirkung auf ein Subjekt, umfassend:
Verwenden von ausgeatmeter Luft, die von einem Subjekt gesammelt wurde, welches die Zusammensetzung gemäß irgendeinem der Ansprüche 1 bis 5 oral genommen hat, als Probe, vor und nach dem Verabreichen der Verdauungstraktmedizin an das Subjekt; und
Vergleichen der Mengen des markierten CO₂, das in die ausgeatmete Luft ausgeschieden wurde, vor und nach der Verabreichung der Verdauungstraktmedizin.

13. Messverfahren gemäß Anspruch 12, worin die Verdauungstraktmedizin die motorische Funktion des Verdauungstrakts betrifft.

14. Messverfahren gemäß Anspruch 12, welches verwendet wird, um die pharmazeutische Wirkung einer Verdauungstraktmedizin oder die therapeutische Wirkung auf einen Patienten, der unter Verdauungsstörungen, die durch eine unzureichende Magenentleerungsfunktion verursacht werden, leidet, zu messen.

15. Verwendung einer Pyrimidinverbindung, ausgewählt aus Uracil und Thymin, zur Herstellung eines diagnostischen Mittels für Verdauungsstörungen, worin die Pyrimidinverbindung mit entweder einem oder beiden Isotopen von C und O markiert ist, die im Körper zu Isotop-markiertem CO₂-gas umgewandelt werden und dann in ausgeatmete Luft ausgeschieden werden.

## Revendications

1. Composition pour une utilisation dans un procédé de mesure de la fonction de vidange gastrique comprenant au moins un composé de pyrimidine choisi parmi l'uracile et la thymine comme principe actif,
le composé de pyrimidine étant marqué avec au moins un élément choisi dans le groupe constitué de ¹³C, ¹⁴C et ¹⁸O, converti en CO₂ marqué dans le corps, et excrété dans l'air expiré.

2. Composition selon la revendication 1, dans laquelle la composition contient des supports et/ou des additifs pharmaceutiquement acceptables quelconques.

3. Composition selon la revendication 2, dans laquelle la composition est produite sous forme solide choisie dans le groupe constitué de comprimé, de comprimé à mâcher, de capsule, de pilule, de poudres, de fines particules et de granules.

4. Composition pour une utilisation dans un procédé de diagnostic de la dyspepsie, où la composition est constituée d'une composition telle que définie dans l'une quelconque des revendications 1 et 3.

5. Composition selon la revendication 4, dans laquelle la dyspepsie est provoquée par une fonction de vidange gastrique insuffisante.

6. Procédé de mesure de la fonction de vidange gastrique comprenant le fait :
d'utiliser l'air expiré prélevé chez un sujet à qui une composition de l'une quelconque des revendications 1 à 3 a été administrée par voie orale, en tant qu'échantillon d'essai ; et
de mesurer *in vitro* la quantité du CO₂ marqué dans l'échantillon d'essai.

7. Procédé de mesure de la fonction de vidange gastrique selon la revendication 6, qui comprend le fait :
d'utiliser l'air expiré provenant d'un sujet chez qui une baisse ou une accélération de la fonction de vidange gastrique est suspectée et à qui une composition de l'une quelconque des revendications 1 à 3 a été administrée par voie orale, en tant qu'échantillon d'essai ;
de mesurer *in vitro* la quantité du CO₂ marqué dans l'échantillon d'essai ; et
de comparer la valeur mesurée avec la quantité du CO₂ marqué dans l'air expiré prélevé chez un sujet sain à qui la même composition a été administrée.

8. Procédé de diagnostic de la dyspepsie comprenant le fait :
d'utiliser l'air expiré prélevé chez un sujet à qui la composition de la revendication 4 ou 5 a été administrée par voie orale, en tant qu'échantillon d'essai ;
de mesurer *in vitro* la quantité du CO₂ marqué dans l'échantillon d'essai ; et
de mesurer la fonction de vidange gastrique.

9. Procédé de diagnostic de la dyspepsie selon la revendication 8, qui comprend le fait :
d'utiliser l'air expiré prélevé chez un sujet chez qui la dyspepsie est suspectée et à qui la composition de la revendication 4 ou 5 a été administrée par voie orale, en tant qu'échantillon d'essai ;
de mesurer *in vitro* la quantité du CO₂ marqué dans l'échantillon d'essai ;
de comparer la valeur mesurée avec la quantité de CO₂ marqué dans l'air expiré prélevé chez un sujet sain à qui la même composition a été administrée ; et
de mesurer la fonction de vidange gastrique.

10. Procédé de diagnostic de la dyspepsie selon la revendication 8 ou 9, dans lequel la dyspepsie est provoquée par une fonction de vidange gastrique insuffisante.

11. Procédé de mesure d'un effet de traitement gastro-intestinal sur un sujet comprenant le fait :
d'utiliser, en tant qu'échantillon d'essai, l'air expiré prélevé chez un sujet à qui la composition de l'une quelconque des revendications 1 à 5 a été administrée par voie orale, avant et après la mise en oeuvre du traitement gastro-intestinal ; et
de comparer les quantités du CO₂ marqué excrété dans l'air expiré avant et après le traitement gastro-intestinal.

12. Procédé d'évaluation d'un effet pharmaceutique d'un médicament gastro-intestinal ou d'un effet thérapeutique sur un sujet comprenant le fait :
d'utiliser, en tant qu'échantillon d'essai, l'air expiré prélevé chez un sujet à qui la composition de l'une quelconque des revendications 1 à 5 a été administrée par voie orale, avant et après l'administration du médicament gastro-intestinal au sujet ; et
de comparer les quantités du CO₂ marqué excrété dans l'air expiré avant et après l'administration du médicament gastro-intestinal.

13. Procédé de mesure selon la revendication 12, dans lequel le médicament gastro-intestinal affecte la fonction motrice de tube digestif.

14. Procédé de mesure selon la revendication 12, qui est utilisé pour mesurer l'effet pharmaceutique d'un médicament gastro-intestinal ou l'effet thérapeutique sur un patient souffrant de la dyspepsie provoquée par une fonction de vidange gastrique insuffisante.

15. Utilisation d'un composé de pyrimidine choisi parmi l'uracile et la thymine pour produire un agent de diagnostic de la dyspepsie, le composé de pyrimidine étant marqué avec un isotope de C ou/et un isotope de O, converti en un gaz CO₂ marqué avec un isotope dans le corps, et excrété ensuite dans l'air expiré.
